(19) Europäisches Patentamt
European Patent Office
Office européen des brevets

(11) **EP 4 424 669 A1**

(12) **EUROPEAN PATENT APPLICATION**
published in accordance with Art. 153(4) EPC

(43) Date of publication:
**04.09.2024 Bulletin 2024/36**

(21) Application number: **22886074.8**

(22) Date of filing: **27.10.2022**

(51) International Patent Classification (IPC):
***C07D 201/16*** (2006.01)   ***C07D 223/10*** (2006.01)

(52) Cooperative Patent Classification (CPC):
**C07D 201/04; C07D 201/16; C07D 223/10;**
**Y02P 20/52**

(86) International application number:
**PCT/CN2022/127978**

(87) International publication number:
**WO 2023/072199 (04.05.2023 Gazette 2023/18)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **27.10.2021 CN 202111258077**

(71) Applicants:
• **China Petroleum & Chemical Corporation**
**Beijing 100728 (CN)**
• **Sinopec Research Institute of Petroleum**
**Processing Co., Ltd.**
**Beijing 100083 (CN)**

(72) Inventors:
• **FAN, Yingqi**
**Beijing 100083 (CN)**

• **WANG, Hao**
**Beijing 100083 (CN)**
• **LUO, Yibin**
**Beijing 100083 (CN)**
• **XIE, Li**
**Beijing 100083 (CN)**
• **ZHANG, Xiaoxin**
**Beijing 100083 (CN)**
• **ZHANG, Dejiang**
**Beijing 100083 (CN)**
• **LI, Qiang**
**Beijing 100083 (CN)**

(74) Representative: **Hoffmann Eitle**
**Patent- und Rechtsanwälte PartmbB**
**Arabellastraße 30**
**81925 München (DE)**

(54) **METHOD FOR PRODUCING HIGH-PURITY EPSILON-CAPROLACTAM**

(57) The present invention relates to the field of caprolactam production, and discloses a process for producing high-purity ε-caprolactam. The process comprises the following steps: (1) cyclohexanone oxime is subjected to a gas phase Beckmann rearrangement reaction; (2) the reaction product obtained from step (1) is successively subjected to gas-liquid separation, solvent removal, and light impurity removal to produce a crude caprolactam; (3) the crude caprolactam is subjected to crystallization to produce a crystallization product and a crystallization mother liquor; (4) the crystallization mother liquor is subjected to crystallization to produce a crystal slurry; at least a part of the crystal slurry is returned to step (2) and/or step (3). The purification process of the present invention can obtain high-purity caprolactam through only one crystallization and one mother liquor crystallization without reducing the overall yield of caprolactam. In addition, the process provided by the present invention can effectively solve the problem of product quality fluctuation.

Figure 2

**Description**

**Technical Field**

The present invention relates to the field of caprolactam production, and specifically relates to a process for producing high-purity ε-caprolactam.

**Background of Art**

Caprolactam is one of the important raw materials for synthetic fibers and synthetic resins. It is mainly used to manufacture polyamide fibers (nylon 6), resins and films, etc. Known processes for producing caprolactam include the liquid-phase Beckmann rearrangement of cyclohexanone oxime using fuming sulfuric acid as a catalyst, the gas-phase Beckmann rearrangement of cyclohexanone oxime using solid zeolite as a catalyst, depolymerization of waste polymers, and the like. The gas-phase Beckmann rearrangement reaction of cyclohexanone oxime on a solid acid catalyst is a new process to achieve the production of caprolactam without ammonium sulfate. It has no problems such as equipment corrosion and environmental pollution. The separation and purification of the product will also be greatly simplified. Therefore the new gas-phase Beckmann rearrangement reaction process without ammonium sulfate has received great attention from people in the industry.

However, the caprolactam obtained by these processes contains various impurities. As well known, caprolactam is a raw material used to prepare polyamide. Caprolactam products used to prepare polyamide and further manufacture synthetic fibres and synthetic resins are required to be of high quality. Impurities at the μg/g level will affect the subsequent polymerization of caprolactam, resulting in difficulties in forming filaments. Therefore, it is necessary to use various separation and purification processes to obtain crude caprolactam, and then use various refining processs to finally obtain high-purity caprolactam, so that high-purity caprolactam can be used to manufacture products such as synthetic fibers, synthetic resins, and films. Known purification processes for the gas phase rearrangement product caprolactam include distillation, crystallization, hydrogenation and the like.

Although the above purification processes may be effective in producing high-purity caprolactam, actual industrial applications do face the problems of how to increase the caprolactam yield of the entire gas phase rearrangement process and improve the adaptability of the refinement system to the reaction system, while obtaining high-purity ε-caprolactam.

**Summary of the Invention**

The object of the present invention is to provide a process for producing high-purity ε-caprolactam, in order to overcome the problems existing in the prior art of unstable caprolactam product quality (low purity) and low caprolactam yield in the production process. The process provided in the present invention can effectively solve the problem of product quality fluctuation and obtain high-purity caprolactam without reducing the overall yield of caprolactam.

In order to achieve the above objects, the present invention provides a process for producing high-purity ε-caprolactam, the process comprises the following steps:

(1) cyclohexanone oxime is subjected to a gas phase Beckmann rearrangement reaction;
(2) the reaction product obtained from step (1) is successively subjected to gas-liquid separation, solvent removal, and light impurity removal to produce a crude caprolactam;
(3) the crude caprolactam is subjected to crystallization to produce a crystallization product and a crystallization mother liquor;
(4) the crystallization mother liquor is subjected to crystallization to produce a crystal slurry;

at least a part of the crystal slurry is returned to step (2) and/or step (3).

The purification process of the present invention can obtain high-purity caprolactam through only one crystallization and one mother liquor crystallization without reducing the overall yield of caprolactam. In addition, the process provided by the present invention can effectively solve the problem of product quality fluctuation.

Specifically, the present invention provides the following technical solutions:

1. A process for producing high-purity ε-caprolactam, which process comprises the following steps:

(2) a reaction product obtained from a reaction that forms ε-caprolactam is treated to obtain a crude caprolactam, the treating includes one or more of neutralization (such as liquid ammonia neutralization), extraction (such as ammonium sulfate extraction and benzene extraction), stripping, gas-liquid separation, solvent removal, water removal, light impurity removal and heavy impurity removal;

(3) the crude caprolactam is subjected to crystallization to produce a crystallization product and a crystallization mother liquor;

(4) the crystallization mother liquor is subjected to crystallization to produce a crystal slurry, and at least a part of the crystal slurry is subjected to countercurrent washing to produce a caprolactam-rich crystal slurry and a caprolactam-lean stream;

at least a part of the caprolactam-rich crystal slurry is returned to step (2) and/or step (3).

2. A process for producing high-purity ε-caprolactam, which process comprises the following steps:

(1) cyclohexanone oxime is subjected to a gas phase Beckmann rearrangement reaction;
(2) the reaction product obtained from step (1) is successively subjected to gas-liquid separation, solvent removal, and light impurity removal to produce a crude caprolactam;
(3) the crude caprolactam is subjected to crystallization to produce a crystallization product and a crystallization mother liquor;
(4) the crystallization mother liquor is subjected to crystallization to produce a crystal slurry;

at least a part of the crystal slurry is returned to step (2) and/or step (3).

3. A process for producing high-purity ε-caprolactam, which process comprises the following steps:

(1) cyclohexanone oxime is subjected to a gas phase Beckmann rearrangement reaction;
(2) the reaction product obtained from step (1) is successively subjected to gas-liquid separation, solvent removal, optionally water removal, and light impurity removal to produce a crude caprolactam;
(3) the crude caprolactam is subjected to crystallization to produce a crystallization product and a crystallization mother liquor;
(4) the crystallization mother liquor is subjected to crystallization to produce a crystal slurry, and at least a part of the crystal slurry is subjected to countercurrent washing to produce a caprolactam-rich crystal slurry and a caprolactam-lean stream;

at least a part of the caprolactam-rich crystal slurry is returned to step (2) and/or step (3).

4. The process according to any one of the preceding technical solutions, wherein the process comprises the crystal slurry is subjected to countercurrent washing,

the countercurrent washing comprises the crystal slurry and a washing solvent are subjected to countercurrent contact in a washing apparatus, the washing apparatus is provided with at least one dense phase bed of caprolactam crystal,
optionally, the washing apparatus is provided with at least one dilute phase bed of caprolactam crystal;
preferably, the dense phase bed of caprolactam crystal has a bed particle density of 400-1000 kg/m$^3$, preferably 500-900 kg/m$^3$;
preferably, the dilute phase bed of caprolactam crystal has a bed particle density of 100-500 kg/m$^3$, preferably 100-400 kg/m$^3$;
more preferably, the bed particle density of the dense phase bed of caprolactam crystal is greater than the bed particle density of the dilute phase bed of caprolactam crystal.

5. The process according to any one of the preceding technical solutions, wherein the crystal slurry is sent to the washing apparatus from the upper part of the washing apparatus, and the washing solvent is sent to the washing apparatus from the lower part of the washing apparatus.

6. The process according to any one of the preceding technical solutions, wherein a dense phase bed of caprolactam crystal and a dilute phase bed of caprolactam crystal are successively arranged in the washing apparatus from top to bottom.

7. The process according to any one of the preceding technical solutions, wherein the process does not comprise a step of heavy impurity removal in step (2); and/or the process does not comprise hydrogenation before obtaining high-purity ε-caprolactam (for example, purity>_99.99 wt%).

8. The process according to any one of the preceding technical solutions, wherein at leaat a part of the crystal slurry is returned to step (2) to perform the solvent removal.

9. The process according to any one of the preceding technical solutions, wherein wherein the amount of the crystal slurry returned to step (2) and/or step (3) comprises 2-50 wt%, preferably 5-30 wt% of the crude caprolactam.

10. The process according to any one of the preceding technical solutions, wherein the process further comprises

the crystal slurry is subjected to countercurrent washing, then the caprolactam crystal obtained from washing and the solvent are subjected to water phase dissolution and layer separation to produce an aqueous caprolactam solution, then the aqueous caprolactam solution is returned to a location after gas-liquid separation and solvent removal of step (2) to perform the water removal, and then perform the light impurity removal;

preferably, the used amount of the water phase is 0.05-5 times, preferably 0.1-1 times the mass of the caprolactam crystal obtained from washing;

preferably, the water phase dissolution and layer separation is dissolution at normal temperature or dissolution under heating, the temperature for the dissolution at normal temperature is 10-30°C, the temperature for the dissolution under heating is 30-110°C.

11. The process according to any one of the preceding technical solutions, wherein the process further comprises the crystal slurry is subjected to countercurrent washing, then the caprolactam crystal obtained from washing and the solvent are subjected to dissolution under heating to produce a caprolactam solution, then the caprolactam solution is returned to step (3) to perform the crystallization.

12. The process according to any one of the preceding technical solutions, wherein the countercurrent washing comprises: the crystal slurry and a washing solvent are subjected to countercurrent contact in a washing apparatus, wherein the crystal slurry is sent to the washing apparatus from the upper part of the washing apparatus, the washing solvent is sent to the washing apparatus from the lower part of the washing apparatus;

wherein a dense phase bed of caprolactam crystal and a dilute phase bed of caprolactam crystal are successively arranged in the washing apparatus from top to bottom;

preferably, the dense phase bed of caprolactam crystal has a bed particle density of 400-1000 $kg/m^3$, preferably 500-900 $kg/m^3$;

preferably, the dilute phase bed of caprolactam crystal has a bed particle density of 100-500 $kg/m^3$, preferably 100-400 $kg/m^3$;

more preferably, the bed particle density of the dense phase bed of caprolactam crystal is greater than the bed particle density of the dilute phase bed of caprolactam crystal.

13. The process according to any one of the preceding technical solutions, wherein based on the total height of the dense phase bed of caprolactam crystal and the dilute phase bed of caprolactam crystal, the height of the dense phase bed of caprolactam crystal is 50-80%, the height of the dilute phase bed of caprolactam crystal is 20-50%; preferably, based on the total height of the dense phase bed of caprolactam crystal and the dilute phase bed of caprolactam crystal, the height of the dense phase bed of caprolactam crystal is 50-70%, the height of the dilute phase bed of caprolactam crystal is 30-50%.

14. The process according to any one of the preceding technical solutions, wherein the introduction rate of the washing solvent is 0.001-0.2 m/s, further preferably 0.005-0.15 m/s;

preferably, the used amount of the washing solvent is 0.1-10 times, preferably 0.2-5 times, more preferably 0.2-2 times the mass of caprolactam crystal in the crystal slurry;

preferably, the washing solvent is at least one of halohydrocarbon (such as halo$C_{1-6}$alkane, halo is fluoro, chloro, bromo, or iodo), ether (such as $C_2$-$C_{12}$alkyl mono- or poly-ether, $C_2$-$C_{12}$alkenyl mono- or poly-ether) and alkane having a carbon atom number of 6-12;

preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane).

15. The process according to any one of the preceding technical solutions, wherein step (1) comprises cyclohexanone oxime is subjected to the gas phase Beckmann rearrangement reaction in the presence of solvent and carrier gas;

preferably, in step (1), the cyclohexanone oxime is reacted on a solid acid catalyst, the solid acid catalyst contains Ti-Si molecular sieve, all-silicon molecular sieve or molecular sieve having an MFI structure;

preferably, in step (1), the solvent is $C_1$-$C_6$ fatty alcohol, preferably at least one of methanol, ethanol and propanol;

preferably, in step (1) the carrier gas is at least one of nitrogen gas, hydrogen gas, argon gas, ammonia gas and saturated hydrocarbons and halohydrocarbons having a boiling point of not higher than 180°C.

16. The process according to any one of the preceding technical solutions (except for technical solution 7), wherein step (2) further comprises performing heavy impurity removal after light impurity removal.

17. An plant for producing high-purity ε-caprolactam, the plant comprises:

an operation unit (2) for treating a reaction product obtained from a reaction that forms ε-caprolactam to produce a crude caprolactam;
an operation unit (3) for subjecting the crude caprolactam to crystallization to produce a crystallization product and a crystallization mother liquor;
an operation unit (4) for subjecting the crystallization mother liquor to crystallization to produce a crystal slurry; wherein the operation unit (4) comprises an equipment for subjecting the crystal slurry to countercurrent washing, the countercurrent washing equipment is provided with a dense phase bed of ε-caprolactam crystal and/or internal component(s) for forming the dense phase bed such as partition(s) or valve(s);
an equipment for returning at leaat a part of the washed crystal slurry from the operation unit (4) to the operation unit (2) and/or the operation unit (3).

18. The plant for producing high-purity ε-caprolactam according to any one of the preceding technical solutions, wherein the countercurrent washing equipment is further provided with a dilute phase bed of ε-caprolactam crystal.

19. The plant for producing high-purity ε-caprolactam according to any one of the preceding technical solutions, wherein the amount of the dense phase bed of ε-caprolactam crystal is at least one, for example, 1, 2, 3, 4, 5 or 6; the amount of the dilute phase bed of ε-caprolactam crystal is 0 or at least one, for example 0, 1, 2, 3, 4, 5 or 6.

20. The plant for producing high-purity ε-caprolactam according to any one of the preceding technical solutions, wherein the bed particle density of the dense phase bed of ε-caprolactam crystal is 400-1000 $kg/m^3$, preferably 500-900 $kg/m^3$;

preferably, the dilute phase bed of caprolactam crystal has a bed particle density of 100-500 $kg/m^3$, preferably 100-400 $kg/m^3$;
more preferably, the bed particle density of the dense phase bed of caprolactam crystal is greater than the bed particle density of the dilute phase bed of caprolactam crystal.

21. The plant for producing high-purity ε-caprolactam according to any one of the preceding technical solutions, wherein based on the total height of the dense phase bed of caprolactam crystal and the dilute phase bed of caprolactam crystal, the height of the dense phase bed of caprolactam crystal is 50-80%, the height of the dilute phase bed of caprolactam crystal is 20-50%;
preferably, based on the total height of the dense phase bed of caprolactam crystal and the dilute phase bed of caprolactam crystal, the height of the dense phase bed of caprolactam crystal is 50-70%, the height of the dilute phase bed of caprolactam crystal is 30-50%.

22. The plant for producing high-purity ε-caprolactam according to any one of the preceding technical solutions, wherein the operation unit (2) comprises: one or more of neutralization (such as liquid ammonia neutralization) equipment, extraction (such as ammonium sulfate extraction and benzene extraction) equipment, steam stripping equipment, gas-liquid separation equipment, solvent removal equipment, water removal equipment, light impurity removal equipment, and heavy impurity removal equipment.

23. The plant for producing high-purity ε-caprolactam according to any one of the preceding technical solutions, wherein the plant does not include an heavy impurity removal equipment.

24. A process for countercurrent washing an organic crystal slurry, wherein

the organic crystal slurry comprises crystallizable compound, impurity, and organic solvent;
the method of countercurrent washing comprises the crystal slurry and a washing solvent are subjected to countercurrent contact in a washing apparatus, the washing apparatus is provided with at least one dense phase bed of the crystal of crystallizable compound;
the washing apparatus is optionally provided with at least one dilute phase bed of the crystal of crystallizable compound.

25. The process for countercurrent washing an organic crystal slurry according to any one of the preceding technical solutions, wherein

the organic solvent is at least one of halohydrocarbon (such as halo$C_{1-6}$alkane, halo is fluoro, chloro, bromo, or iodo), ether (such as $C_2$-$C_{12}$alkyl mono- or poly-ether, $C_2$-$C_{12}$alkenyl mono- or poly-ether) and alkane having a carbon atom number of 6-12; preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane);

the concentration of the crystallizable compound in the organic crystal slurry is sufficient to separate out the crystallizable compound in crystal form, for example, it can be 10-90 wt%, for example 20-70 wt%, 30-45 wt%, or 35-40 wt%.

In the above technical solution, the crystallizable compound includes but is not limited to lactam compounds; and the impurity refers to all compounds except the crystallizable compound and the organic solvent.

26. The process for countercurrent washing an organic crystal slurry according to any one of the preceding technical solutions, wherein the crystal slurry is sent to the washing apparatus from the upper part of the washing apparatus, the washing solvent is sent to the washing apparatus from the lower part of the washing apparatus;
preferably, a dense phase bed of the crystal of crystallizable compound and a dilute phase bed of the crystal of crystallizable compound are successively arranged in the washing apparatus from top to bottom.

27. The process for countercurrent washing an organic crystal slurry according to any one of the preceding technical solutions, wherein

the crystal slurry is sent to the washing apparatus from a crystal slurry inlet, and separated crystals move downward under the action of gravity and form a dilute/dense phase bed of the crystal of crystallizable compound in the washing apparatus;
the washing solvent is sent to the washing apparatus from a washing solvent inlet, a part of the washing solvent flows upwards through the dilute/dense phase bed of the crystal of crystallizable compound to displace the crystallization mother liquor, this part of the washing solvent is discharged together with the crystallization mother liquor from a mother liquor outlet, and the remaining washing solvent is carried along with the crystal and discharged.

28. The process for countercurrent washing an organic crystal slurry according to any one of the preceding technical solutions, wherein

the dense phase bed of the crystal of crystallizable compound has a bed particle density of 400-1000 kg/m$^3$, preferably 500-900 kg/m$^3$; and/or
the dilute phase bed of the crystal of crystallizable compound has a bed particle density of 100-500 kg/m$^3$, preferably 100-400 kg/m$^3$;
the bed particle density of the dense phase bed of the crystal of crystallizable compound is greater than the bed particle density of the dilute phase bed of the crystal of crystallizable compound.

29. The process for countercurrent washing an organic crystal slurry according to any one of the preceding technical solutions, wherein

to the washing apparatus is loaded the crystal of crystallizable compound to form a dilute phase bed of the crystal of crystallizable compound or a dense phase bed of the crystal of crystallizable compound; and/or
a crystal slurry is added to an upper part of the washing apparatus, a washing solvent is added to a lower part of the washing apparatus, so that the crystal slurry and the washing solvent are subjected to countercurrent contact during the washing to separate out the crystal, then a dilute phase bed of the crystal of crystallizable compound and a dense phase bed of the crystal of crystallizable compound are formed in the washing apparatus by controlling the moving-downward velocity of the crystal in the washing apparatus.

30. The process for countercurrent washing an organic crystal slurry according to any one of the preceding technical solutions, wherein
partition(s) or valve(s) is/are arranged in the washing apparatus. The partition or valve is used to reduce the flow cross-sectional area of the crystallizable compound to form the dense phase bed of the crystal of crystallizable compound and the dilute phase bed of the crystal of crystallizable compound.

31. The process for countercurrent washing an organic crystal slurry according to any one of the preceding technical solutions, wherein

> based on the total height of the dense phase bed of the crystal of crystallizable compound and the dilute phase bed of the crystal of crystallizable compound, the height of the dense phase bed of the crystal of crystallizable compound is 50-80%, the height of the dilute phase bed of the crystal of crystallizable compound is 20-50%; and/or
> based on the total height of the dense phase bed of the crystal of crystallizable compound and the dilute phase bed of the crystal of crystallizable compound, the height of the dense phase bed of the crystal of crystallizable compound is 50-70%, the height of the dilute phase bed of the crystal of crystallizable compound is 30-50%.

32. The process for countercurrent washing an organic crystal slurry according to any one of the preceding technical solutions, wherein
the purity of the crystal of crystallizable compound in the dense phase bed of the crystal of crystallizable compound and that in the dilute phase bed of the crystal of crystallizable compound are each independently 98.0-99.9 wt%.

33. The process for countercurrent washing an organic crystal slurry according to any one of the preceding technical solutions, wherein
the introduction rate of the washing solvent is 0.001-0.2 m/s, further preferably 0.005-0.15 m/s; the used amount of the washing solvent is 0.1-10 times, preferably 0.2-5 times, more preferably 0.2-2 times the mass of the crystal of crystallizable compound in the crystal slurry.

34. The process for countercurrent washing an organic crystal slurry according to any one of the preceding technical solutions, wherein
the washing solvent is at least one of halohydrocarbon (such as $haloC_{1-6}$alkane, halo is fluoro, chloro, bromo, or iodo), ether (such as $C_2$-$C_{12}$alkyl mono- or poly-ether, $C_2$-$C_{12}$alkenyl mono- or poly-ether) and alkane having a carbon atom number of 6-12; preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane).

35. The process for countercurrent washing an organic crystal slurry according to any one of the preceding technical solutions, wherein
The dense phase bed of the crystal of crystallizable compound is stirred, preferably, the dense phase bed of the crystal of crystallizable compound is stirred with paddle(s) or partition(s) driven by an electric machine.

36. The process for countercurrent washing an organic crystal slurry according to any one of the preceding technical solutions, wherein the crystallizable compound is ε-caprolactam, preferably ε-caprolactam obtained by subjecting cyclohexanone oxime to a gas phase Beckmann rearrangement reaction.

## Description of the drawings

Figure 1 is a schematic diagram of a valve provided in a washing apparatus according to an embodiment of the process provided by the present invention;
Figure 2 is a schematic diagram of a partition provided in a washing apparatus according to an embodiment of the process provided by the present invention;
Figure 3 is a schematic diagram of an plant and process for producing high-purity ε-caprolactam of the present invention.
Figure 4 is a schematic diagram of another plant and process for producing high-purity ε-caprolactam of the present invention.
Figure 5 is a schematic diagram of yet another plant and process for producing high-purity ε-caprolactam of the present invention.

Explanation of reference signs

a--------crystal slurry inlet

b--------washing solvent inlet

c--------mother liquor outlet

d--------(aqueous) caprolactam solution outlet

e--------circulating water inlet

f--------heating pipe

g--------valve

h--------partition

2--------operation unit for treating a reaction product obtained from a reaction that forms ε-caprolactam to produce a crude caprolactam

3--------operation unit for subjecting the crude caprolactam to crystallization to produce a crystallization product and a crystallization mother liquor

4--------operation unit for subjecting the crystallization mother liquor to crystallization to produce a crystal slurry

**Detailed description**

The endpoints of ranges and any values disclosed herein are not limited to the precise ranges or values, but these ranges or values are to be understood to include values approaching such ranges or values. Moreover, for the numerical ranges, one or more new numerical ranges can be obtained by the combinations between any two endpoint values of the ranges, between an endpoint value of the ranges and a specific point value, and between any two specific point values. These numerical ranges shall be deemed to be specifically disclosed herein.

In the present invention, the pressure refers to gauge pressure.

In the present invention, caprolactam and ε-caprolactam are synonymous.

In the present invention, bed particle density = total mass of crystallizable compound (such as caprolactam) particles in the bed/total volume of the bed.

In the present invention, "dilute phase bed of caprolactam crystal" and "dilute phase bed" are interchangeable, "dense phase bed of caprolactam crystal" and "dense phase bed" are interchangeable, "crystal slurry containing caprolactam crystal and crystallization mother liquor" and "crystal slurry" are interchangeable, "caprolactam crystal" and "crystal" are interchangeable.

In the description of the present invention, it should be understood that the orientation or positional relationship indicated by the terms "top/upper", "bottom/lower", etc. is based on the orientation or positional relationship shown in the drawings, and is only for the convenience of describing the present invention and simplifying the description. It is not intended to indicate or imply that the apparatus or element referred to must have a specific orientation, be constructed and operated in a specific orientation, and therefore is not to be construed as a limitation of the invention. In addition, "inside/in and outside" refer to the inside and outside with respect to the outline of each component itself.

In the present invention, the "purity of caprolactam" refers to the content as the percentage of the weight of caprolactam relative to the total weight of caprolactam and impurity, wherein the impurity does not include the washing solvent and the crystallization solvent in the mother liquor.

The high purity mentioned in the present invention means that the purity of ε-caprolactam is not less than 99.99 wt%.

In the present invention, the "particle density" refers to the bulk density of crystal particles, specifically the weight of the crystal of crystallizable compound (e.g., caprolactam) per unit volume.

The present invention provides a process for countercurrent washing an organic crystal slurry, wherein the organic crystal slurry comprises crystallizable compound, impurity, and organic solvent; the organic solvent may be at least one of halohydrocarbon (such as halo$C_{1-6}$alkane, halo is fluoro, chloro, bromo, or iodo), ether (such as $C_2$-$C_{12}$alkyl mono- or poly-ether, $C_2$-$C_{12}$alkenyl mono- or poly-ether) and alkane having a carbon atom number of 6-12; preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane); the concentration of the crystallizable compound in the organic crystal slurry is sufficient to separate out the crystallizable compound in crystal form, for example, it can be 10-90 wt%, for example 20-70 wt%, 30-45 wt%, or 35-40 wt%;

the method of countercurrent washing comprises the crystal slurry and a washing solvent are subjected to countercurrent contact in a washing apparatus, the washing apparatus is provided with at least one dense phase bed of the crystal of crystallizable compound;
the washing apparatus is optionally provided with at least one dilute phase bed of the crystal of crystallizable compound.

According to an embodiment of the process for countercurrent washing an organic crystal slurry of the present invention, the crystal slurry is sent to the washing apparatus from the upper part of the washing apparatus, and the washing solvent is sent to the washing apparatus from the lower part of the washing apparatus.

According to an embodiment of the process for countercurrent washing an organic crystal slurry of the present invention, a dense phase bed of the crystal of crystallizable compound and a dilute phase bed of the crystal of crystallizable compound are successively arranged in the washing apparatus from top to bottom. According to an embodiment of the process for countercurrent washing an organic crystal slurry of the present invention, the crystal slurry is sent to the washing apparatus from a crystal slurry inlet, and separated crystals move downward under the action of gravity and form a dilute/dense phase bed of the crystal of crystallizable compound in the washing apparatus; the washing solvent is sent to the washing apparatus from a washing solvent inlet, a part of the washing solvent flows upwards through the dilute/dense phase bed of the crystal of crystallizable compound to displace the crystallization mother liquor, this part of the washing solvent is discharged together with the crystallization mother liquor from a mother liquor outlet, and the remaining washing solvent is carried along with the crystal and discharged.

According to an embodiment of the process for countercurrent washing an organic crystal slurry of the present invention, the dense phase bed of the crystal of crystallizable compound has a bed particle density of 400-1000 kg/m$^3$, preferably 500-900 kg/m$^3$; and/or the dilute phase bed of the crystal of crystallizable compound has a bed particle density of 100-500 kg/m$^3$, preferably 100-400 kg/m$^3$; the bed particle density of the dense phase bed of the crystal of crystallizable compound is greater than the bed particle density of the dilute phase bed of the crystal of crystallizable compound.

According to an embodiment of the process for countercurrent washing an organic crystal slurry of the present invention, to the washing apparatus is loaded the crystal of crystallizable compound to form a dilute phase bed of the crystal of crystallizable compound or a dense phase bed of the crystal of crystallizable compound; and/or a crystal slurry is added to an upper part of the washing apparatus, a washing solvent is added to a lower part of the washing apparatus, so that the crystal slurry and the washing solvent are subjected to countercurrent contact during the washing to separate out the crystal, then a dilute phase bed and a dense phase bed are formed in the washing apparatus by controlling the moving-downward velocity of the crystal in the washing apparatus.

According to an embodiment of the process for countercurrent washing an organic crystal slurry of the present invention, partition(s) or valve(s) is/are arranged in the washing apparatus. The partition or valve is used to reduce the flow cross-sectional area of the crystallizable compound to form the dense phase bed of the crystal of crystallizable compound and the dilute phase bed of the crystal of crystallizable compound.

According to an embodiment of the process for countercurrent washing an organic crystal slurry of the present invention, based on the total height of the dense phase bed of the crystal of crystallizable compound and the dilute phase bed of the crystal of crystallizable compound, the height of the dense phase bed of the crystal of crystallizable compound is 50-80%, the height of the dilute phase bed of the crystal of crystallizable compound is 20-50%; and/or based on the total height of the dense phase bed of the crystal of crystallizable compound and the dilute phase bed of the crystal of crystallizable compound, the height of the dense phase bed of the crystal of crystallizable compound is 50-70%, the height of the dilute phase bed of the crystal of crystallizable compound is 30-50%.

According to an embodiment of the process for countercurrent washing an organic crystal slurry of the present invention, the purity of the crystal of crystallizable compound in the dense phase bed of the crystal of crystallizable compound and that in the dilute phase bed of the crystal of crystallizable compound are each independently 98.0-99.9 wt%.

According to an embodiment of the process for countercurrent washing an organic crystal slurry of the present invention, the introduction rate of the washing solvent is 0.001-0.2 m/s, further preferably 0.005-0.15 m/s.

According to an embodiment of the process for countercurrent washing an organic crystal slurry of the present invention, the used amount of the washing solvent is 0.1-10 times, preferably 0.2-5 times, more preferably 0.2-2 times the mass of the crystal of crystallizable compound in the crystal slurry.

According to an embodiment of the process for countercurrent washing an organic crystal slurry of the present invention, the washing solvent may be at least one of halohydrocarbon (such as haloC$_{1-6}$alkane, halo is fluoro, chloro, bromo, or iodo), ether (such as C$_2$-C$_{12}$alkyl mono- or poly-ether, C$_2$-C$_{12}$alkenyl mono- or poly-ether) and alkane having a carbon atom number of 6-12; preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane). According to an embodiment of the process for countercurrent washing an organic crystal slurry of the present invention, the dense phase bed of the crystal of crystallizable compound is stirred, preferably, the dense phase bed of the crystal of crystallizable compound is stirred with paddle(s) or partition(s) driven by an electric machine.

According to an embodiment of the process for countercurrent washing an organic crystal slurry of the present invention, the crystallizable compound is ε-caprolactam, preferably ε-caprolactam obtained by subjecting cyclohexanone oxime to a gas phase Beckmann rearrangement reaction.

The present invention also provides an plant for producing high-purity ε-caprolactam, wherein the plant comprises:

an operation unit (2) for treating a reaction product obtained from a reaction that forms ε-caprolactam to produce a crude caprolactam;
an operation unit (3) for subjecting the crude caprolactam to crystallization to produce a crystallization product and a crystallization mother liquor;
an operation unit (4) for subjecting the crystallization mother liquor to crystallization to produce a crystal slurry;
wherein the operation unit (4) comprises an equipment for subjecting the crystal slurry to countercurrent washing, the countercurrent washing equipment is provided with a dense phase bed of ε-caprolactam crystal and/or internal component(s) for forming the dense phase bed such as partition(s) or valve(s).

According to an embodiment of the plant for producing high-purity ε-caprolactam of the present invention, the plant further comprises an equipment for returning at leaat a part of the washed crystal slurry from the operation unit (4) to the operation unit (2) and/or the operation unit (3).

According to an embodiment of the plant for producing high-purity ε-caprolactam of the present invention, the countercurrent washing equipment is further provided with a dilute phase bed of ε-caprolactam crystal.

The amount of the dense phase bed of ε-caprolactam crystal can be at least one, for example, 1, 2, 3, 4, 5 or 6. The amount of the dilute phase bed of ε-caprolactam crystal can be 0 or at least one, for example 0, 1, 2, 3, 4, 5 or 6. The bed particle density of the dense phase bed of ε-caprolactam crystal can be 400-1000 kg/m$^3$, preferably 500-900 kg/m$^3$; the bed particle density of the dilute phase bed of caprolactam crystal can be 100-500 kg/m$^3$, preferably 100-400 kg/m$^3$; the bed particle density of the dense phase bed of caprolactam crystal is greater than the bed particle density of the dilute phase bed of caprolactam crystal, based on the total height of the dense phase bed of caprolactam crystal and the dilute phase bed of caprolactam crystal, the height of the dense phase bed of caprolactam crystal can be 50-80%, the height of the dilute phase bed of caprolactam crystal can be 20-50%; preferably, based on the total height of the dense phase bed of caprolactam crystal and the dilute phase bed of caprolactam crystal, the height of the dense phase bed of caprolactam crystal can be 50-70%, the height of the dilute phase bed of caprolactam crystal can be 30-50%.

According to an embodiment of the plant for producing high-purity ε-caprolactam of the present invention, the operation unit (2) comprises: one or more of neutralization (such as liquid ammonia neutralization) equipment, extraction (such as ammonium sulfate extraction and benzene extraction) equipment, steam stripping equipment, gas-liquid separation equipment, solvent removal equipment, water removal equipment, light impurity removal equipment, and heavy impurity removal equipment.

According to an embodiment of the plant for producing high-purity ε-caprolactam of the present invention, the operation unit (2) does not include an heavy impurity removal equipment.

In the present invention, relative to ε-caprolactam, the light impurity is an impurity having a boiling point less than that of ε-caprolactam, the heavy impurity is an impurity having a boiling point greater than that of ε-caprolactam.

The present invention also provides a process for producing high-purity ε-caprolactam, which process comprises the following steps:

(2) a reaction product obtained from a reaction that forms ε-caprolactam is treated to obtain a crude caprolactam, the treating includes one or more of neutralization (such as liquid ammonia neutralization), extraction (such as ammonium sulfate extraction and benzene extraction), stripping, gas-liquid separation, solvent removal, water removal, light impurity removal and heavy impurity removal;
(3) the crude caprolactam is subjected to crystallization to produce a crystallization product and a crystallization mother liquor;
(4) the crystallization mother liquor is subjected to crystallization to produce a crystal slurry, and at least a part of the crystal slurry is subjected to countercurrent washing to produce a caprolactam-rich crystal slurry and a caprolactam-lean stream;
at least a part of the caprolactam-rich crystal slurry is returned to step (2) and/or step (3).

The present invention also provides a process for producing high-purity ε-caprolactam, which process comprises the following steps:

(1) cyclohexanone oxime is subjected to a gas phase Beckmann rearrangement reaction;

(2) the reaction product obtained from step (1) is successively subjected to gas-liquid separation, solvent removal, and light impurity removal to produce a crude caprolactam;

(3) the crude caprolactam is subjected to crystallization to produce a crystallization product and a crystallization mother liquor;

(4) the crystallization mother liquor is subjected to crystallization to produce a crystal slurry;

at least a part of the crystal slurry is returned to step (2) and/or step (3).

The present invention also provides a process for producing high-purity ε-caprolactam, which process comprises the following steps:

(1) cyclohexanone oxime is subjected to a gas phase Beckmann rearrangement reaction;

(2) the reaction product obtained from step (1) is successively subjected to gas-liquid separation, solvent removal, optionally water removal, and light impurity removal to produce a crude caprolactam;

(3) the crude caprolactam is subjected to crystallization to produce a crystallization product and a crystallization mother liquor;

(4) the crystallization mother liquor is subjected to crystallization to produce a crystal slurry, and at least a part of the crystal slurry is subjected to countercurrent washing to produce a caprolactam-rich crystal slurry and a caprolactam-lean stream;

at least a part of the caprolactam-rich crystal slurry is returned to step (2) and/or step (3).

According to the present invention, the countercurrent washing comprises: the crystal slurry and a washing solvent are subjected to countercurrent contact in a washing apparatus, the washing apparatus is provided with at least one dense phase bed of caprolactam crystal, optionally, the washing apparatus is provided with at least one dilute phase bed of caprolactam crystal.

According to an embodiment of the present invention, the crystal slurry is sent to the washing apparatus from the upper part of the washing apparatus, and the washing solvent is sent to the washing apparatus from the lower part of the washing apparatus.

According to an embodiment of the present invention, a dense phase bed of caprolactam crystal and a dilute phase bed of caprolactam crystal are successively arranged in the washing apparatus from top to bottom.

According to an embodiment of the present invention, with respect to the process for producing high-purity ε-caprolactam, the process does not comprise a step of heavy impurity removal in step (2); and/or the process does not comprise hydrogenation before obtaining high-purity ε-caprolactam (for example, purity>_99.99 wt%).

In the present invention, the gas-phase Beckmann rearrangement reaction in step (1) can be carried out according to the conventional manners in the art. The present invention has no special limitations thereon. In order to better illustrate the present invention, the illustrative explanation of a specific gas-phase Beckmann rearrangement reaction will be provided hereinafter.

According to the present invention, preferably, step (1) comprises cyclohexanone oxime is subjected to the gas phase Beckmann rearrangement reaction in the presence of solvent and carrier gas.

According to the present invention, preferably, the weight ratio of solvent to cyclohexanone oxime is 1:1 3:1.

According to the present invention, preferably, in step (1), the solvent is $C_1$-$C_6$ fatty alcohol, preferably at least one of methanol, ethanol and propanol.

According to the present invention, the carrier gas can be any gas that does not react with cyclohexanone oxime and the solvent in the condition of the gas phase Beckmann rearrangement reaction, preferably, in step (1), the carrier gas is at least one of nitrogen gas, hydrogen gas, argon gas, ammonia gas and saturated hydrocarbon (for example methanol, ethanol, hexane, cyclohexane) and halohydrocarbon (for example 1-chloropropane, 2-chloropropane, chloro-n-butane, 2-chlorobutane) having a boiling point of not higher than 180°C.

According to the present invention, the gas phase Beckmann rearrangement reaction can be carried out according to the conventional technologies in the art. The present invention has no special limitations thereon. Preferably, the conditions of the gas phase Beckmann rearrangement reaction comprise: the temperature is 320-450°C, preferably 330-400°C; the pressure is 0.05-1.0 MPa, preferably 0.2-0.5 MPa; the weight hourly space velocity of cyclohexanone oxime is 0.1-5 h$^{-1}$.

According to the present invention, the catalyst used in the gas phase Beckmann rearrangement reaction can be those catalysts commonly used in the art, but in order to increase the yield of crude caprolactam, preferably in step (1), the cyclohexanone oxime is reacted on a solid acid catalyst, the solid acid catalyst contains Ti-Si molecular sieve, all-silicon molecular sieve or molecular sieve having an MFI structure.

According to the present invention, in order to increase the purity of crude caprolactam, step (2) further comprises performing heavy impurity removal after light impurity removal.

**EP 4 424 669 A1**

According to the present invention, in step (2), conditions of gas-liquid separation, solvent removal, light impurity removal and heavy impurity removal can be selected in a relatively wide range. The present invention has no particular limitation on the condition of gas-liquid separation, as long as the purpose of gas-liquid separation can be achieved. The solvent removal is to remove the solvent used in the gas phase Beckmann rearrangement reaction. Those skilled in the art can select appropriate solvent removal methods and conditions according to the type of specific solvent, which will not be described in detail here.

The light impurity removal in the present invention refers to the removal of impurities with a boiling point lower than caprolactam. Specifically, the method of vacuum distillation can be used. The heavy impurity removal in the present invention refers to the removal of impurities with a boiling point higher than caprolactam.

Preferably, the conditions of gas-liquid separation, solvent removal, light impurity removal and heavy impurity removal in step (2) can be such those that the purity of the obtained crude caprolactam is 98.8-99.7 wt%.

According to the present invention, in step (3), the conditions of subjecting the crude caprolactam to crystallization can be selected in a relatively wide range, preferably, the conditions of subjecting the crude caprolactam to crystallization comprise: the crystallization temperature is 10-60°C, the crystallization pressure is 0.1-1.5 bar (absolute pressure).

In step (3), the solvent used for subjecting the crude caprolactam to crystallization is an organic solvent, for example at least one of halohydrocarbon (such as $haloC_{1-6}alkane$, halo is fluoro, chloro, bromo, or iodo), ether (such as $C_2$-$C_{12}alkyl$ mono- or poly-ether, $C_2$-$C_{12}alkenyl$ mono- or poly-ether) and alkane having a carbon atom number of 6-12; preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane).

According to the present invention, in step (4), the conditions of subjecting the crystallization mother liquor to crystallization can be those conditions commonly used in the art, as long as a crystal separates out of the mother liquor to form a crystal slurry, preferably, the conditions of subjecting the crystallization mother liquor to crystallization comprise: the crystallization temperature is 10-60°C, the crystallization pressure is 0.1-1.5 bar (absolute pressure), more preferably, the conditions of subjecting the crystallization mother liquor to crystallization are such those that in the crystal slurry, the mass content of caprolactam is 10-90 wt%, for example 20-70 wt%, 30-45 wt%, or 35-40 wt%.

In step (4), the solvent used for subjecting the crystallization mother liquor to crystallization is an organic solvent, for example at least one of halohydrocarbon (such as $haloC_{1-6}alkane$, halo is fluoro, chloro, bromo, or iodo), ether (such as $C_2$-$C_{12}alkyl$ mono- or poly-ether, $C_2$-$C_{12}alkenyl$ mono- or poly-ether) and alkane having a carbon atom number of 6-12; preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane).

According to the present invention, the crystallizer used for subjecting the crude caprolactam to crystallization in step (3) and the crystallizer used for subjecting the crystallization mother liquor to crystallization in step (4) are not particularly limited, and can be a cooling-type crystallizer, an evaporation crystallizer and a vacuum-type crystallizer, and may include at least one of a forced external circulation-type crystallizer, an Oslo-type crystallizer, an FC-type crystallizer, a DTB-type crystallizer, a DP-type crystallizer and a Messo turbulence crystallizer. According to the present invention, preferably, at leaat a part of the crystal slurry is returned to step (2) to perform the solvent removal.

According to the present invention, the amount of the crystal slurry returned to step (2) and/or step (3) can be selected in a relatively wide range. In order to increase the yield of ε-caprolactam while ensuring the purity of ε-caprolactam, preferably, the amount of the crystal slurry returned to step (2) and/or step (3) comprises 2-50 wt%, preferably 5-30 wt% of the crude caprolactam.

According to the present invention, preferably, the process further comprises the crystal slurry is subjected to countercurrent washing, then the caprolactam crystal obtained from washing and the solvent are subjected to water phase dissolution and layer separation to produce an aqueous caprolactam solution, then the aqueous caprolactam solution is returned to a location after gas-liquid separation and solvent removal of step (2) to perform the water removal, and then perform the light impurity removal. Adopting this preferred embodiment is more conducive to obtaining high-purity ε-caprolactam and at the same time improving the yield of caprolactam in the entire process.

According to the present invention, the water phase dissolution and layer separation can be performed according to conventional technical means in the art. Preferably, the used amount of the water phase is 0.05-5 times, preferably 0.1-1 times the mass of the caprolactam crystal obtained from washing. The water phase can be desalted water, such as deionized water.

According to the present invention, preferably, the water phase dissolution and layer separation is dissolution at normal temperature or dissolution under heating, the temperature for the dissolution at normal temperature is 10-30°C, the temperature for the dissolution under heating is 30-110°C.

According to the present invention, preferably, the process further comprises the crystal slurry is subjected to countercurrent washing, then the caprolactam crystal obtained from washing and the solvent are subjected to dissolution under heating (the temperature for dissolution under heating is 50-110°C) to produce a caprolactam solution, then the caprolactam solution is returned to step (3) to perform the crystallization. Adopting this preferred embodiment is more conducive to obtaining high-purity ε-caprolactam and at the same time improving the yield of caprolactam in the entire process.

According to the present invention, preferably, the countercurrent washing comprises: the crystal slurry and a washing solvent are subjected to countercurrent contact in a washing apparatus, wherein the crystal slurry is sent to the washing apparatus from the upper part of the washing apparatus, the washing solvent is sent to the washing apparatus from the lower part of the washing apparatus;

wherein a dense phase bed of caprolactam crystal and a dilute phase bed of caprolactam crystal are successively arranged in the washing apparatus from top to bottom.

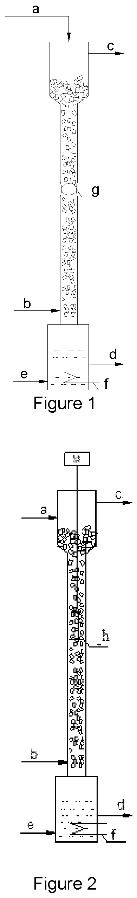

In the present invention, "countercurrent contact" refers to that the crystal slurry is sent to the washing apparatus from a crystal slurry inlet, and separated crystals move downward under the action of gravity and form a dilute/dense phase bed of caprolactam crystal in the washing apparatus; and at the same time, the washing solvent is sent to the washing apparatus from a washing solvent inlet, a part of the washing solvent flows upwards through the dilute/dense phase bed of caprolactam crystal to displace the crystallization mother liquor, this part of the washing solvent is discharged together with the crystallization mother liquor from a mother liquor outlet, and the remaining washing solvent is carried along with the crystal and discharged. The "countercurrent contact" of the present invention is further explained in conjunction with Figure 1. The washing apparatus includes a crystal slurry inlet a, a washing solvent inlet b, a mother liquor outlet c, an (aqueous) caprolactam solution outlet d, a circulating water inlet e, a heating pipe f and a valve g. The crystal slurry is sent to the washing apparatus from the crystal slurry inlet a, and the washing solvent is sent to the washing apparatus from the washing solvent inlet b. The crystal slurry and the washing solvent are subjected to countercurrent washing in the washing apparatus. The mother liquor in the crystal slurry is discharged from the mother liquor outlet c, and the separated crystal moves downward under the action of gravity, and forms a dense phase bed of caprolactam crystal above the valve g and a dilute phase bed of caprolactam crystal below the valve g. The circulating water is introduced from the circulating water inlet e, the dissolution is under the action of the heating pipe f to produce a caprolactam solution, which is discharged from the (aqueous) caprolactam solution outlet d.

In the present invention, in order to describe the position where the crystal slurry and the washing solvent are sent to the washing apparatus, the expressions "upper part" and "lower part" are introduced. The expression "upper part" and "lower part" are comparatively speaking and do not refer to a specific position point or area. For example, the top of the washing apparatus can be called the "upper part" of the bottom of the washing apparatus, and conversely, the bottom of the washing apparatus can be called the "lower part" of the top of the washing apparatus; again for example, the position at half the height of the washing apparatus can be called the "lower part" of the top of the washing apparatus and can also be called the "upper part" at the bottom of the washing apparatus. Meanwhile, the "upper part" and the "lower part" can be adjacent regions, for example, "the region between the top of the washing apparatus and the position at half the height of the washing apparatus" can be called the "upper part" of "the region between the bottom of the washing apparatus and the position at half the height of the washing apparatus" and conversely, "the region between the bottom of the washing apparatus and the position at half the height of the washing apparatus" can be called the "lower part" of "the region between the top of the washing apparatus and the position at half the height of the washing apparatus". The "upper part" and the "lower part" can also be non-adjacent regions for example, "the region between the top of the washing apparatus and the position at one third the height of the washing apparatus" can be called the "upper part" of "the region between the bottom of the washing apparatus and the position at half the height of the washing apparatus" and conversely, "the region between the bottom of the washing apparatus and the position at half the height of the washing apparatus" can be called the "lower part" of "the region between the top of the washing apparatus and the position at one third the height of the washing apparatus" In the present invention, it is generally said that the dense phase bed of caprolactam crystal is located at the upper part of the dilute phase bed of caprolactam crystal. Correspondingly, the dilute phase bed of caprolactam crystal is located at the lower part of the dense phase bed of caprolactam crystal.

According to the present invention, preferably, the dense phase bed of caprolactam crystal has a bed particle density of 400-1000 $kg/m^3$ preferably 500-900 $kg/m^3$

According to the present invention, preferably, the dilute phase bed of caprolactam crystal has a bed particle density of 100-500 $kg/m^3$ preferably 100-400 $kg/m^3$

According to the present invention, the moving-downward velocity of the crystal can be selected within a relatively wide range, preferably as long as the bed particle density of the dense phase bed and/or the dilute phase bed can meet the above ranges.

In the present invention, there is no particular limitation on the formation method of the dilute phase bed of caprolactam crystal and the dense phase bed of caprolactam crystal in the washing apparatus, as long as the above characteristics are met, the object of the present invention can be achieved. For example, caprolactam crystal can be loaded to the washing apparatus before the start-up to form a dilute phase bed of caprolactam crystal or a dense phase bed of caprolactam crystal; it can also be formed during the process operation. Specifically, a crystal slurry may be directly added to an upper part of the washing apparatus, a washing solvent may be added to a lower part of the washing apparatus, so that the crystal slurry and the washing solvent are subjected to countercurrent contact during the washing to separate out the crystal, then a dilute phase bed and a dense phase bed may be formed in the washing apparatus by controlling the moving-downward velocity of the crystal in the washing apparatus.

According to the present invention, preferably, partition(s) (as shown in Figure 2) or valve(s) is/are arranged in the washing apparatus. The partition or valve is used to reduce the flow cross-sectional area of caprolactam to form the dense phase bed of caprolactam crystal and the dilute phase bed of caprolactam crystal. The present invention has no particular limitation on the arrangement of the partition or the valve, as long as the above purpose can be achieved. It is preferred that the partition or the valve is located at the dividing line between the dense phase bed of caprolactam crystal and the dilute phase bed of caprolactam crystal in the washing apparatus.

According to the present invention, preferably, based on the total height of the dense phase bed of caprolactam crystal and the dilute phase bed of caprolactam crystal, the height of the dense phase bed of caprolactam crystal is 50-80% the height of the dilute phase bed of caprolactam crystal is 20-50% more preferably, based on the total height of the dense phase bed of caprolactam crystal and the dilute phase bed of caprolactam crystal the height of the dense phase bed of caprolactam crystal is 50-70% the height of the dilute phase bed of caprolactam crystal is 30-50%

According to the present invention, preferably, the purity of caprolactam crystal in the dense phase bed of caprolactam crystal and that in the dilute phase bed of caprolactam crystal are each independently 98.0-99.9 wt%.

According to the present invention, preferably, the introduction rate of the washing solvent is 0.001-0.2 m/s further preferably 0.005-0.15 m/s; adopting this preferred embodiment is more conducive to cleaning the crystal surface, and the upward-flowing washing solvent may entrain the crystal as few as possible.

According to the present invention, the used amount of the washing solvent can be selected in a relatively wide range, but in order to reduce the discharge of waste liquid and reduce production cost on the premise of ensuring the washing effect, preferably, the used amount of the washing solvent is 0.1-10 times, preferably 0.2-5 times, more preferably 0.2-2 times the mass of caprolactam crystal in the crystal slurry.

According to the present invention, the residence time of caprolactam crystal in the washing apparatus may be selected in a relatively wide range, but taking into account the purity and yield of the crystal and the used amount of washing solvent, preferably the residence time of caprolactam crystal in the washing apparatus is 400-600 seconds.

According to the present invention, the washing solvent can be a commonly used solvent for the crystallization of caprolactam. However, in order to further improve the purity of caprolactam crystal, the washing efficiency, and the yield of caprolactam, preferably, the washing solvent can be at least one of halohydrocarbon (such as halo$C_{1-6}$alkane halo is fluoro, chloro, bromo, or iodo) ether (such as $C_2$-$C_{12}$alkyl mono- or poly-ether, $C_2$-$C_{12}$alkenyl mono- or poly-ether) and alkane having a carbon atom number of 6-12; more preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether diethyl ether n-propyl ether isopropyl ether n-butyl ether ethyl butyl ether ethylene glycol dimethyl ether vinyl ether methyl tert-butyl ether and ethyl tert-butyl ether the alkane having a carbon atom number of 6-12 has a boiling point of 60-180°C (for example, n-heptane n-hexane isopentane n-octane n-nonane methylhexane isohexane neohexane isoheptane isooctane isononane) preferably 60-130°C (for example, pentane n-hexane isopentane).

According to the present invention, preferably, the process further comprises stirring the dense phase bed of caprolactam crystal; adopting this preferred embodiment is more conducive to the movement of the crystal bed on the one hand, and can more effectively wash the crystal surface on the other hand.

According to the present invention, preferably, the dense phase bed of caprolactam crystal is stirred with paddle(s) or partition(s) driven by an electric machine.

According to the present invention, preferably, the upper part of the washing apparatus is provided with an overflow port, and the washed mother liquor flows out of the washing apparatus from the overflow port. Moreover, a part of the washing solvent that flows upward also flows out from the overflow port.

The plant and process of the present invention will be briefly described below with reference to the accompanying drawings.

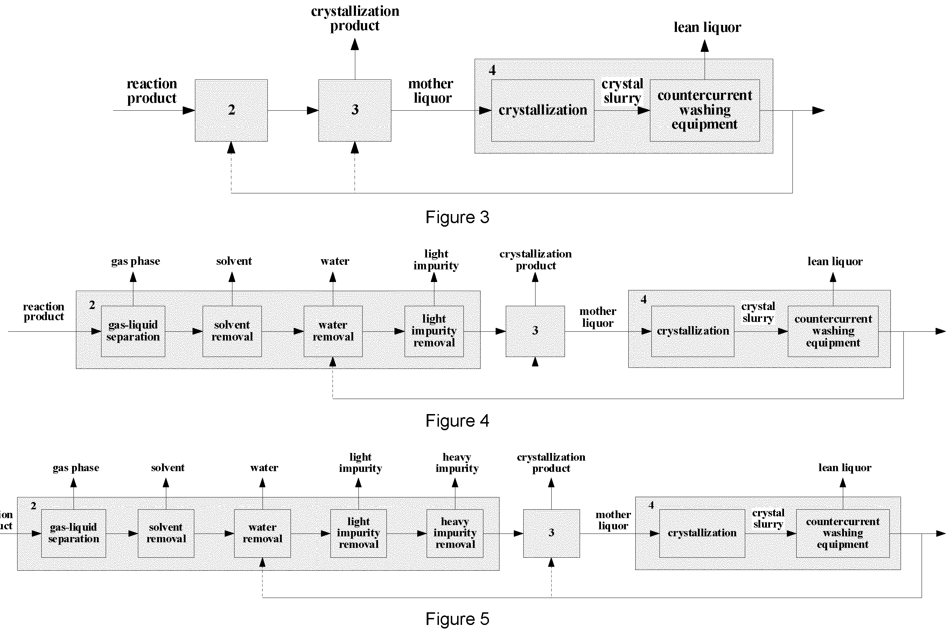

Figure 3 illustrates an plant and process for producing high-purity ε-caprolactam of the present invention. The plant comprises: an operation unit (2) for treating a reaction product obtained from a reaction that forms ε-caprolactam to produce a crude caprolactam; an operation unit (3) for subjecting the crude caprolactam to crystallization to produce a crystallization product and a crystallization mother liquor; an operation unit (4) for subjecting the crystallization mother liquor to crystallization to produce a crystal slurry According to the present invention, there is no particular limitation on the reaction that forms ε-caprolactam. It can be the liquid-phase Beckmann rearrangement of cyclohexanone oxime using fuming sulfuric acid as a catalyst, the gas-phase Beckmann rearrangement of cyclohexanone oxime using solid zeolite as a catalyst, depolymerization of waste polymers, and the like. These methods are known and will not be described in detail herein. The operation unit (2) may include one or more of neutralization (such as liquid ammonia neutralization) equipment extraction (such as ammonium sulfate extraction and benzene extraction) equipment steam stripping equipment gas-liquid separation equipment solvent removal equipment water removal equipment light impurity removal equipment and heavy impurity removal equipment. Finally a crude caprolactam stream is obtained. The purity of caprolactam in the crude caprolactam stream can reach 98.8-99.7 wt%. The operation unit (3) comprises crystallization equipment, and the operation unit (4) comprises crystallization equipment and countercurrent washing equipment. The present invention has no particular limitation on crystallization equipments in the operation units (3) and (4), which may be a cooling-type crystallizer, an evaporation crystallizer and a vacuum crystallizer, and may include at least one of a forced external circulation-type crystallizer, an Oslo-type crystallizer, an FC-type crystallizer, a DTB-type crystallizer, a DP-type crystallizer and a Messo turbulence crystallizer. In the counter-current washing equipment of the operating unit (4), the crystal slurry from the crystallization equipment may be subjected to countercurrent washing. The countercurrent washing equipment is provided with a dense phase bed of ε-caprolactam crystal and/or internal component(s) for forming the dense phase bed such as partition(s) or valve(s). The plant may also comprise an equipment for returning at leaat a part of the washed crystal slurry from the operation unit (4) to the operation unit (2) and/or the operation unit (3) such as pump(s) and pipeline(s). The process for producing high-purity ε-caprolactam of the present invention can be performed by using the plant.

Figure 4 illustrates a specific plant and process for producing high-purity ε-caprolactam of the present invention. The plant and process in Figure 4 are identical to those illustrated in Figure 3 except that in the operation unit (2), the gas-liquid separation equipment, the solvent removal equipment, the water removal equipment (optional), and the light impurity removal equipment that are sequentially connected are used to ultimately produce a crude caprolactam stream. At leaat a part of the washed crystal slurry from the operation unit (4) is returned to the water removal equipment of the operation unit (2) and/or the operation unit (3).

Figure 5 illustrates another specific plant and process for producing high-purity ε-caprolactam of the present invention. The plant and process in Figure 5 are identical to those illustrated in Figure 4 except that in the operation unit (2), the gas-liquid separation equipment, the solvent removal equipment, the water removal equipment (optional), the light impurity removal equipment, and the heavy impurity removal equipment that are sequentially connected are used to ultimately produce a crude caprolactam stream.

The following test methods were used to evaluate the quality of the prepared caprolactam products in the following examples:

(1) purity of caprolactam
A capillary column Innowax60m and a gas chromatography 7890GC were used to analyze the caprolactam purity and the impurity content. The minimum detection limit of chromatography was 1 μg/g.
(2) potassium permanganate (PM) absorption value of ε-caprolactam
3.000 grams of caprolactam was poured into a 100 mL colourimetric tube. Distilled water was added and diluted to the scale. The tube was shaken well, and placed in a constant temperature water bath at 20°C. 1 mL of 0.01N potassium permanganate solution was added into the colourimetric tube and the resulting mixture was shaken well immediately. At the same time, the stopwatch was started. When the colour of the sample solution in the colourimetric tube became the same as the colour of the standard colourimetric solution (which was prepared by dissolving 3 grams of superior-grade pure $Co(NO_3)_2 \cdot 6H_2O$ and 12 mg of superior-grade pure $K_2Cr_2O_7$ in water, diluting to 1 litre, and shaking the content well), the stopwatch was stopped and the consumed time (in seconds) was recorded, which was the absorption value of potassium permanganate.
(3) volatile base (V.B)
In an alkaline medium, the alkaline low-molecular impurities in the sample were distilled out and absorbed with a known amount of hydrochloric acid solution. The excess hydrochloric acid was neutralized by titrating with a sodium hydroxide standard solution. The mole number of the acid consumption per kilogram of the sample was used as the measurement value for volatile base. The calculation formula is as follows:

$$\text{V.B (mmol/kg)} = [(V_0 - V) \times C_{NaOH}/M] \times 1000$$

In the formula: $V_0$ was the volume of NaOH standard solution consumed in the blank test, in mL;

V was the volume of NaOH standard solution consumed by the sample, in mL; $C_{NaOH}$ was the precise concentration of NaOH standard solution, in mol/L;

M was the sample mass, in g.

(4) Extinction value E (at 290nm wavelength)

In a 300ml conical flask, 50 grams of the sample was weighed and 50mL of distilled water was added. The mixture was shaken to completely dissolve the sample, and then allowed to stand for 10 minutes. A spectrophotometer was used to detect the extinction value of a sample with a concentration of 50 wt% relative to distilled water at a wavelength of 290 nm.

(5) The yield of caprolactam product = the mass of the crystallization product obtained instep (3)/the mass of caprolactam in the reaction product of step (2)×100%.

The present invention will be described in detail below through examples.

Example 1

This example was carried out in the manner shown in Figure 5 in combination with Figure 1, in which the washed caprolactam-rich crystal slurry was subjected to water phase dissolution and layer separation, and then returned to the water removal equipment of the operation unit (2).

(1) cyclohexanone oxime was subjected to a gas phase Beckmann rearrangement reaction in the presence of nitrogen gas, solvent methanol and all-silica molecular sieve, wherein the weight ratio of solvent to cyclohexanone oxime was 2:1 the conditions of the gas phase Beckmann rearrangement reaction comprised: the temperature was 350°C, the pressure was 0.4 MPa the weight hourly space velocity of cyclohexanone oxime was 2 h$^{-1}$.

(2) the product of the gas phase Beckmann rearrangement reaction was subjected to cooling followed by gas-liquid separation, the gas phase was compressed and recycled to the reaction system, the liquid phase was subjected to methanol separation, light impurity removal and heavy impurity removal to produce a crude caprolactam with the purity of 99.6 wt%.

(3) the crude caprolactam was subjected to crystallization to produce a crystallization product and a crystallization mother liquor, the crystallization conditions comprised: crystallization temperature: 35°C, crystallization pressure: 1 bar (absolute pressure). The crystallization product was washed and post-treated to produce a caprolactam product.

(4) the crystallization mother liquor was subjected to crystallization to produce a crystal slurry, the crystallization conditions comprised: crystallization temperature: 30°C, crystallization pressure: 1 bar (absolute pressure) the mass content of caprolactam in the crystal slurry was 35 wt% The obtained whole crystal slurry was sent to the washing apparatus to perform the countercurrent washing. Then at 30°C, the caprolactam crystal obtained from washing and the solvent were subjected to water phase dissolution and layer separation (the used amount of the water phase was 0.2 times the mass of the caprolactam crystal obtained from washing) to produce an aqueous caprolactam solution. Then the aqueous caprolactam solution was returned to a location after gas-liquid separation and solvent removal of step (2) to perform the water removal, and then perform the light impurity removal, and the subsequent steps. At the same time, the ratio of the amount of the crude caprolactam to the amount of the crystal slurry returned to step (2) was controlled to 100:20.

Among them, the countercurrent washing process included: the crystal slurry was slowly sent by pump to the washing apparatus from the upper part of the washing apparatus, the washing solvent isopropyl ether (its used amount was 0.5 times the mass of the crystal slurry) was sent to the washing apparatus from the lower part of the washing apparatus. The introduction rate of washing solvent was 0.02 m/s. The crystal slurry and the washing solvent were subjected to countercurrent contact in the washing apparatus to wash caprolactam crystal. The moving-downward velocity of the crystal generated in the washing process was controlled by a value. The dense phase bed of caprolactam crystal was formed in the upper part of the washing apparatus. The dilute phase bed of caprolactam crystal was formed in the lower part of the washing apparatus. A partition was arranged between the dense phase bed of caprolactam crystal and the dilute phase bed of caprolactam crystal. The height ratio of the dense phase bed to the dilute phase bed was 5:5. The height of the dilute phase bed was 50% of the height of the washing apparatus. The particle density in the dense phase bed was 850 kg/m$^3$. The particle density in the dilute phase bed was 400 kg/m$^3$. At the same time the residue time of caprolactam crystal in the washing apparatus was controlled to 480s.

Caprolactam product was obtained in step (3), yield: 99.0 wt% purity: 99.997 wt% (mass fraction) extinction value: 0.02 volatile base value: 0.05 PM value: 38000s colourity: not greater than 1. The product indicators were all better than premium grade standards of China National Standard.

Example 2

This example was carried out in the manner shown in Figure 4 in combination with Figure 2, in which the washed caprolactam-rich crystal slurry was subjected to dissolution under heating, and then returned to the operation unit (3).

(1) cyclohexanone oxime was subjected to a gas phase Beckmann rearrangement reaction in the presence of nitrogen gas, solvent methanol and all-silica molecular sieve, wherein the weight ratio of solvent to cyclohexanone oxime was 2:1 the conditions of the gas phase Beckmann rearrangement reaction comprised: the temperature was 350°C, the pressure was 0.4 MPa the weight hourly space velocity of cyclohexanone oxime was 2 h$^{-1}$.

(2) the product of the gas phase Beckmann rearrangement reaction was subjected to cooling followed by gas-liquid separation, the gas phase was compressed and recycled to the reaction system, the liquid phase was subjected to methanol separation, light impurity removal and heavy impurity removal to produce a crude caprolactam with the purity of 99 wt%.

(3) the crude caprolactam was subjected to crystallization to produce a crystallization product and a crystallization mother liquor, the crystallization conditions comprised: crystallization temperature: 35°C, crystallization pressure: 1 bar (absolute pressure). The crystallization product was washed and post-treated to produce a caprolactam product.

(4) the crystallization mother liquor was subjected to crystallization to produce a crystal slurry, the crystallization conditions comprised crystallization temperature: 30°C, crystallization pressure: 1 bar (absolute pressure) the mass content of caprolactam in the crystal slurry: 40 wt% The obtained whole crystal slurry was sent to the washing apparatus to perform the countercurrent washing. Then the caprolactam crystal obtained from washing and the solvent were subjected to dissolution under heating (the temperature for dissolution under heating was 70°C) to produce a caprolactam solution. Then the caprolactam solution is returned to step (3) to perform the crystallization. At the same time, the ratio of the amount of the crude caprolactam to the amount of the crystal slurry returned to step (3) was controlled to 100:30.

Among them, the countercurrent washing process included: the crystal slurry was slowly sent by pump to the washing apparatus from the upper part of the washing apparatus, the washing solvent isopropyl ether (its used amount was 0.6 times the mass of the crystal slurry) was sent to the washing apparatus from the lower part of the washing apparatus. The introduction rate of washing solvent was 0.01 m/s. The crystal slurry and the washing solvent were subjected to countercurrent contact in the washing apparatus to wash caprolactam crystal. The moving-downward velocity of the crystal generated in the washing process was controlled by a value. The dense phase bed of caprolactam crystal was formed in the upper part of the washing apparatus. The dilute phase bed of caprolactam crystal was formed in the lower part of the washing apparatus. A partition was arranged between the dense phase bed of caprolactam crystal and the dilute phase bed of caprolactam crystal. The height ratio of the dense phase bed to the dilute phase bed was 6:4. The height of the dilute phase bed was 40% of the height of the washing apparatus. The particle density in the dense phase bed was 820 kg/m$^3$. The particle density in the dilute phase bed was 380 kg/m$^3$. At the same time the residue time of caprolactam crystal in the washing apparatus was controlled to 480s.

Caprolactam product was obtained in step (3), yield: 99.2 wt% purity: 99.995 wt% (mass fraction) extinction value: 0.03 volatile base value: 0.1 PM value: 30000s colourity: not greater than 1. The product indicators were all better than premium grade standards of China National Standard.

It can be seen from Example 1-2 that using the process of the present invention to produce caprolactam can increase the yield of caprolactam product while ensuring the quality of the caprolactam product.

Example 3

Example 3 was carried out in the manner shown in Figure 4 in combination with Figure 1. Example 3 was identical to Example 1, except that in step (2), the product of the gas phase Beckmann rearrangement reaction was subjected to cooling followed by gas-liquid separation, the gas phase was compressed and recycled to the reaction system, the liquid phase was subjected to methanol separation and light impurity removal to produce a crude caprolactam; and the obtained crude caprolactam was treated according to steps (3) and (4) of Example 1.

Example 4

Example 4 was carried out in the manner shown in Figure 5 in combination with Figure 2. Example 4 was identical to Example 2, except that in step (2), the product of the gas phase Beckmann rearrangement reaction was subjected to cooling followed by gas-liquid separation, the gas phase was compressed and recycled to the reaction system, the liquid phase was subjected to methanol separation, light impurity removal, and heavy impurity removal to produce a crude caprolactam; and the obtained crude caprolactam was treated according to steps (3) and (4) of Example 2.

In Examples 3 and 4, the caprolactam products obtained in step (3) had the same quality as the caprolactam products obtained in Example 1 and Example 2 respectively.

It can also be seen from Examples 1-4 that using the process of the present invention to produce caprolactam can increase the yield of caprolactam product while ensuring the quality of the caprolactam product, and can omit the heavy impurity removal equipment and improve the economy of the plant.

The preferred embodiments of the present invention have been described in detail above, but the present invention is not limited thereto. Within the scope of the technical concept of the present invention, various simple modifications can be made to the technical solution of the present invention, including the combination of various technical features in any other suitable manner. These simple modifications and combinations should also be regarded as the disclosed content of the present invention and all belong to the protection scope of the present invention.

## Claims

1. A process for producing high-purity ε-caprolactam, which process comprises the following steps:

    (2) a reaction product obtained from a reaction that forms ε-caprolactam is treated to obtain a crude caprolactam, the treating includes one or more of neutralization (such as liquid ammonia neutralization), extraction (such as ammonium sulfate extraction and benzene extraction), stripping, gas-liquid separation, solvent removal, water removal, light impurity removal and heavy impurity removal;
    (3) the crude caprolactam is subjected to crystallization to produce a crystallization product and a crystallization mother liquor;
    (4) the crystallization mother liquor is subjected to crystallization to produce a crystal slurry, and at least a part of the crystal slurry is subjected to countercurrent washing to produce a caprolactam-rich crystal slurry and a caprolactam-lean stream;
    at least a part of the caprolactam-rich crystal slurry is returned to step (2) and/or step (3).

2. A process for producing high-purity ε-caprolactam, which process comprises the following steps:

    (1) cyclohexanone oxime is subjected to a gas phase Beckmann rearrangement reaction;
    (2) the reaction product obtained from step (1) is successively subjected to gas-liquid separation, solvent removal, and light impurity removal to produce a crude caprolactam;
    (3) the crude caprolactam is subjected to crystallization to produce a crystallization product and a crystallization mother liquor;
    (4) the crystallization mother liquor is subjected to crystallization to produce a crystal slurry;

    at least a part of the crystal slurry is returned to step (2) and/or step (3).

3. A process for producing high-purity ε-caprolactam, which process comprises the following steps:

    (1) cyclohexanone oxime is subjected to a gas phase Beckmann rearrangement reaction;
    (2) the reaction product obtained from step (1) is successively subjected to gas-liquid separation, solvent removal, optionally water removal, and light impurity removal to produce a crude caprolactam;
    (3) the crude caprolactam is subjected to crystallization to produce a crystallization product and a crystallization mother liquor;
    (4) the crystallization mother liquor is subjected to crystallization to produce a crystal slurry, and at least a part of the crystal slurry is subjected to countercurrent washing to produce a caprolactam-rich crystal slurry and a caprolactam-lean stream;

    at least a part of the caprolactam-rich crystal slurry is returned to step (2) and/or step (3).

4. The process according to any one of the preceding claims, wherein the process further comprises: the crystal slurry is subjected to countercurrent washing,

the countercurrent washing comprises the crystal slurry and a washing solvent are subjected to countercurrent contact in a washing apparatus, the washing apparatus is provided with at least one dense phase bed of caprolactam crystal,

optionally, the washing apparatus is provided with at least one dilute phase bed of caprolactam crystal;

preferably, the dense phase bed of caprolactam crystal has a bed particle density of 400-1000 kg/m$^3$, preferably 500-900 kg/m$^3$;

preferably, the dilute phase bed of caprolactam crystal has a bed particle density of 100-500 kg/m$^3$, preferably 100-400 kg/m$^3$; the bed particle density of the dense phase bed of caprolactam crystal is greater than the bed particle density of the dilute phase bed of caprolactam crystal.

5. The process according to any one of the preceding claims, wherein the crystal slurry is sent to the washing apparatus from the upper part of the washing apparatus, and the washing solvent is sent to the washing apparatus from the lower part of the washing apparatus.

6. The process according to any one of the preceding claims, wherein a dense phase bed of caprolactam crystal and a dilute phase bed of caprolactam crystal are successively arranged in the washing apparatus from top to bottom.

7. The process according to any one of the preceding claims, wherein at leaat a part of the crystal slurry is returned to step (2) to perform the solvent removal.

8. The process according to any one of the preceding claims, wherein the amount of the crystal slurry returned to step (2) and/or step (3) comprises 2-50 wt%, preferably 5-30 wt% of the crude caprolactam.

9. The process according to any one of the preceding claims, wherein the process further comprises the crystal slurry is subjected to countercurrent washing, then the caprolactam crystal obtained from washing and the solvent are subjected to water phase dissolution and layer separation to produce an aqueous caprolactam solution, then the aqueous caprolactam solution is returned to a location after gas-liquid separation and solvent removal of step (2) to perform the water removal, and then perform the light impurity removal;

preferably, the used amount of the water phase is 0.05-5 times, preferably 0.1-1 times the mass of the caprolactam crystal obtained from washing;

preferably, the water phase dissolution and layer separation is dissolution at normal temperature or dissolution under heating, the temperature for the dissolution at normal temperature is 10-30°C, the temperature for the dissolution under heating is 30-110°C.

10. The process according to any one of the preceding claims, wherein the process further comprises the crystal slurry is subjected to countercurrent washing, then the caprolactam crystal obtained from washing and the solvent are subjected to dissolution under heating to produce a caprolactam solution, then the caprolactam solution is returned to step (3) to perform the crystallization.

11. The process according to any one of the preceding claims, wherein the countercurrent washing comprises: the crystal slurry and a washing solvent are subjected to countercurrent contact in a washing apparatus, wherein the crystal slurry is sent to the washing apparatus from the upper part of the washing apparatus, the washing solvent is sent to the washing apparatus from the lower part of the washing apparatus;

wherein a dense phase bed of caprolactam crystal and a dilute phase bed of caprolactam crystal are successively arranged in the washing apparatus from top to bottom;

preferably, the dense phase bed of caprolactam crystal has a bed particle density of 400-1000 kg/m$^3$, preferably 500-900 kg/m$^3$;

preferably, the dilute phase bed of caprolactam crystal has a bed particle density of 100-500 kg/m$^3$, preferably 100-400 kg/m$^3$; the bed particle density of the dense phase bed of caprolactam crystal is greater than the bed particle density of the dilute phase bed of caprolactam crystal.

12. The process according to any one of the preceding claims, wherein based on the total height of the dense phase bed of caprolactam crystal and the dilute phase bed of caprolactam crystal, the height of the dense phase bed of caprolactam crystal is 50-80%, the height of the dilute phase bed of caprolactam crystal is 20-50%;

preferably, based on the total height of the dense phase bed of caprolactam crystal and the dilute phase bed of caprolactam crystal, the height of the dense phase bed of caprolactam crystal is 50-70%, the height of the dilute phase bed of caprolactam crystal is 30-50%.

13. The process according to any one of the preceding claims, wherein the introduction rate of the washing solvent is 0.001-0.2 m/s, further preferably 0.005-0.15 m/s;

preferably, the used amount of the washing solvent is 0.1-10 times, preferably 0.2-5 times, more preferably 0.2-2 times the mass of caprolactam crystal in the crystal slurry;

preferably, the washing solvent is at least one of halohydrocarbon (such as halo$C_{1-6}$alkane, halo is fluoro, chloro, bromo, or iodo), ether (such as $C_2$-$C_{12}$alkyl mono- or poly-ether, $C_2$-$C_{12}$alkenyl mono- or poly-ether) and alkane having a carbon atom number of 6-12;

preferably, the halohydrocarbon is at least one of 1-chloropropane, 2-chloropropane, chloro-n-butane, 2-chlorobutane, chloroisobutane, chlorotertbutane, n-bromopropane, bromoisopropane, 1-bromobutane and 2-bromobutane; the ether is at least one of methyl ethyl ether, diethyl ether, n-propyl ether, isopropyl ether, n-butyl ether, ethyl butyl ether, ethylene glycol dimethyl ether, vinyl ether, methyl tert-butyl ether and ethyl tert-butyl ether; the alkane having a carbon atom number of 6-12 has a boiling point of 60-180°C (for example, n-heptane, n-hexane, isopentane, n-octane, n-nonane, methylhexane, isohexane, neohexane, isoheptane, isooctane, isononane), preferably 60-130°C (for example, pentane, n-hexane, isopentane).

14. The process according to any one of the preceding claims, wherein step (1) comprises cyclohexanone oxime is subjected to the gas phase Beckmann rearrangement reaction in the presence of solvent and carrier gas;

preferably, in step (1), the cyclohexanone oxime is reacted on a solid acid catalyst, the solid acid catalyst contains Ti-Si molecular sieve, all-silicon molecular sieve or molecular sieve having an MFI structure;

preferably, in step (1), the solvent is $C_1$-$C_6$ fatty alcohol, preferably at least one of methanol, ethanol and propanol;

preferably, in step (1) the carrier gas is at least one of nitrogen gas, hydrogen gas, argon gas, ammonia gas and saturated hydrocarbons and halohydrocarbons having a boiling point of not higher than 180°C.

15. The process according to any one of the preceding claims, wherein step (2) further comprises the heavy impurity removal is performed after the light impurity removal.

Figure 1

Figure 2

crystallization
product

reaction
product

mother
liquor

lean liquor

4

| 2 | | 3 | | crystallization | crystal slurry | countercurrent washing equipment |

**Figure 3**

gas phase    solvent    water    light impurity    crystallization product    lean liquor

reaction product

2

| gas-liquid separation | solvent removal | water removal | light impurity removal | | 3 | mother liquor | 4 | crystallization | crystal slurry | countercurrent washing equipment |

**Figure 4**

gas phase    solvent    water    light impurity    heavy impurity    crystallization product    lean liquor

ion ıct

2

| gas-liquid separation | solvent removal | water removal | light impurity removal | heavy impurity removal | | 3 | mother liquor | 4 | crystallization | crystal slurry | countercurrent washing equipment |

**Figure 5**

## INTERNATIONAL SEARCH REPORT

International application No.

**PCT/CN2022/127978**

**A.    CLASSIFICATION OF SUBJECT MATTER**

C07D 201/16(2006.01)i;  C07D 223/10(2006.01)i

According to International Patent Classification (IPC) or to both national classification and IPC

**B.    FIELDS SEARCHED**

Minimum documentation searched (classification system followed by classification symbols)

C07D

Documentation searched other than minimum documentation to the extent that such documents are included in the fields searched

Electronic data base consulted during the international search (name of data base and, where practicable, search terms used)

CNTXT; ENTXTC; VEN: CNKI: 中石化, 中国石油化工, 石油化工科学研究院, 范瑛琦, 己内酰胺, 母液, 结晶, 晶体, 晶浆, 逆流, 洗, 密度, 颗粒, 稀相, 密相, caprolactam, crystal+, slurry, counter current, wash+, density, particle, dilute phase, dense phase,

**C.    DOCUMENTS CONSIDERED TO BE RELEVANT**

| Category* | Citation of document, with indication, where appropriate, of the relevant passages | Relevant to claim No. |
|---|---|---|
| PX | CN 114907263 A (CHINA PETROLEUM & CHEMICAL CORPORATION et al.) 16 August 2022 (2022-08-16) claims 1-9, and description, paragraphs 0002 and 0014-0049 | 1-15 |
| PX | CN 114907264 A (CHINA PETROLEUM & CHEMICAL CORPORATION et al.) 16 August 2022 (2022-08-16) claims 1-9, and description, paragraphs 0014-0049 | 1-15 |
| X | CN 104059019 A (CHINA PETROLEUM & CHEMICAL CORPORATION et al.) 24 September 2014 (2014-09-24) claims 1-15, description, paragraphs 0002 and 0034-0040, embodiments 1 and 4, and figures 2-4 | 1-15 |
| X | CN 104059018 A (CHINA PETROLEUM & CHEMICAL CORPORATION et al.) 24 September 2014 (2014-09-24) claims 1-14, description, paragraphs 0002, 0031-0036, and 0067, and figures 1-4 | 1-15 |
| X | CN 104072419 A (CHINA PETROLEUM & CHEMICAL CORPORATION et al.) 01 October 2014 (2014-10-01) claims 1-15, description, paragraphs 003 and 0059, and figures 1-4 | 1-15 |

☑ Further documents are listed in the continuation of Box C.   ☑ See patent family annex.

| | |
|---|---|
| *     Special categories of cited documents: | "T"    later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"    document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"    document member of the same patent family |

| Date of the actual completion of the international search | Date of mailing of the international search report |
|---|---|
| **23 November 2022** | **30 November 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
|---|
| **PCT/CN2022/127978** |

**C.    DOCUMENTS CONSIDERED TO BE RELEVANT**

| Category* | Citation of document, with indication, where appropriate, of the relevant passages | Relevant to claim No. |
|---|---|---|
| A | CN 207375980 U (CHINA PETROLEUM & CHEMICAL CORPORATION et al.) 18 May 2018 (2018-05-18)<br>    entire document | 1-15 |
| A | US 2002010329 A1 (SUMITOMO CHEMICAL CO., LTD.) 24 January 2002 (2002-01-24)<br>    entire document | 1-15 |
| A | US 2012330006 A1 (NAGAMI HIDETO) 27 December 2012 (2012-12-27)<br>    entire document | 1-15 |

Form PCT/ISA/210 (second sheet) (January 2015)

**INTERNATIONAL SEARCH REPORT**
Information on patent family members

International application No.

**PCT/CN2022/127978**

| Patent document cited in search report | | | Publication date (day/month/year) | Patent family member(s) | | | Publication date (day/month/year) |
|---|---|---|---|---|---|---|---|
| CN | 114907263 | A | 16 August 2022 | None | | | |
| CN | 114907264 | A | 16 August 2022 | None | | | |
| CN | 104059019 | A | 24 September 2014 | CN | 104059019 | B | 25 January 2017 |
| CN | 104059018 | A | 24 September 2014 | CN | 104059018 | B | 26 October 2016 |
| CN | 104072419 | A | 01 October 2014 | CN | 104072419 | B | 02 November 2016 |
| CN | 207375980 | U | 18 May 2018 | None | | | |
| US | 2002010329 | A1 | 24 January 2002 | EP | 1167350 | A1 | 02 January 2002 |
| | | | | ES | 2342044 | T3 | 01 July 2010 |
| | | | | JP | 2002003472 | A | 09 January 2002 |
| | | | | TW | 593276 | B | 21 June 2004 |
| | | | | DE | 60141788 | D1 | 27 May 2010 |
| | | | | CN | 1332158 | A | 23 January 2002 |
| | | | | KR | 20020001633 | A | 09 January 2002 |
| US | 2012330006 | A1 | 27 December 2012 | CN | 102781913 | A | 14 November 2012 |
| | | | | KR | 20130036199 | A | 11 April 2013 |
| | | | | EP | 2542523 | A1 | 09 January 2013 |
| | | | | SG | 183224 | A1 | 27 September 2012 |
| | | | | WO | 2011108251 | A1 | 09 September 2011 |
| | | | | TW | 201202193 | A | 16 January 2012 |
| | | | | JP | 2011201865 | A | 13 October 2011 |

Form PCT/ISA/210 (patent family annex) (January 2015)